# EUROPEAN PATENT APPLICATION

(11) **EP 4 492 391 A1**
(43) Date of publication of application: **15.01.2025**
(21) Application number: 23766884.3
(22) Date of filing: 08.03.2023
(51) Int. Cl.: G16H 20/00, A61B 5/0245, A61B 5/11, A61B 5/16, A61B 5/352

(54) **INFORMATION PROCESSING SYSTEM, INFORMATION PROCESSING METHOD, AND PROGRAM**

(30) Priority: 08.03.2022 JP 2022035010
(71) Applicant: RIKEN, Wako-shi, Saitama 351-0198 (JP)
(72) Inventor: KURODA, Kumi, Wako-shi, Saitama 351-0198 (JP); TAGANE OHMURA, Nami, Wako-shi, Saitama 351-0198 (JP); OKUMA, Lana, Wako-shi, Saitama 351-0198 (JP); ESPOSITO, Gianluca, Wako-shi, Saitama 351-0198 (JP)
(74) Representative: Plasseraud IP
(86) International application number: PCT/JP2023/008805
(87) International publication number: WO 2023/171708

(57) **Abstract**

To attain the object of assisting childcare behaviors for promoting stopping of crying of an infant or sleep of an infant, included are: a sensor information acquisition unit (101) that acquires sensor information from a sensor that detects an infant's state; a calculation unit (103) that calculates, on the basis of the sensor information, at least one of heart rate information (IBI) and an infant state score (ISS); an output unit (104) that outputs, in real time, information indicating at least one of the IBI and the ISS; and a notification unit (105) that outputs notification information for notifying a user of a childcare behavior to be performed to promote stopping of crying of the infant or sleep of the infant on the basis of at least one of the length of time elapsed since start of holding and walking has been detected, the IBI, and the ISS.

## Description

### Technical Field

The present invention relates to a technique for assisting a caregiver on the basis of the state of an infant.

### Background Art

There has been known a technique for assisting a caregiver on the basis of the state of an infant. For example, Patent Literature 1 discloses a system including: a detection means for detecting and recording at least one signal related to oral sucking behavior; and an arrangement means for arranging the detection means in an oral position of an infant, the system, optionally, determining a sleep stage of the infant from recorded data. In addition, Patent Literature 1 discloses, as an example of the arrangement means, a pacifier for use with infants. In this case, the system displays, on a small display which is included in the pacifier, the sleep stage with use of a color code, a number, or the like.

### Citation List

### [Patent Literature]

[Patent Literature 1]
Published Japanese Translation of PCT International Application Tokuhyo No. 2014-530735

### Summary of Invention

### Technical Problem

Here, crying of infants and awakening thereof during the night are major stresses for caregivers. In addition, even after a caregiver has holds and walks with a crying infant until the infant stops crying, and then the caregiver has placed and laid the infant who has stopped crying down to sleep, crying of the infant is often resumed. In such a case, caregiver's stress increases.

In the system disclosed in Patent Literature 1, it is possible to show sleep stages of an infant, but it is difficult for a crying baby to use a pacifier. Thus, stress which a caregiver feels until an infant stops crying or falls asleep is not reduced.

An aspect of the present invention has been attained to solve the above-described problem, and an object thereof is to provide a technique for assisting childcare behaviors for promoting stopping of crying of an infant or promoting sleep of an infant.

### Solution to Problem

In order to solve the above-described problem, an information processing system in accordance with an aspect of the present invention includes: a sensor information acquisition unit that acquires sensor information from a sensor that detects a state of an infant; a calculation unit that calculates, on a basis of the sensor information, at least one selected from the group consisting of heart rate information related to a heart rate of the infant and an infant state score indicating any one of states of the infant from a wailing state to a sleep state; an output unit that outputs, in real time, information indicating at least one selected from the group consisting of the heart rate information and the infant state score; and a notification unit that outputs notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

In order to solve the above-described problem, an information processing method in accordance with an aspect of the present invention includes: a sensor information acquisition step of acquiring sensor information from a sensor that detects a state of an infant; a calculation step of calculating, on a basis of the sensor information, at least one selected from the group consisting of heart rate information related to a heart rate of the infant and an infant state score indicating any one of states of the infant from a wailing state to a sleep state; an output step of outputting, in real time, information indicating at least one selected from the group consisting of the heart rate information and the infant state score; and a notification step of outputting notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

### Advantageous Effects of Invention

According to an aspect of the present invention, it is possible to assist childcare behaviors for promoting stopping of crying of an infant or promoting sleep of an infant.

### Brief Description of Drawings

Fig. 1 is a graph for describing analysis result 1 on an experiment from which the findings that form foundations for the present invention were obtained.
Fig. 2 is a graph for describing analysis result 2 on the above-described experiment.
Fig. 3 is a graph for describing analysis result 3 on the above-described experiment.
Fig. 4 is a block diagram illustrating an example of a hardware configuration of an information processing system in accordance with an embodiment of the present invention.
Fig. 5 is a block diagram illustrating an example of a functional configuration of an information processing system in accordance with an embodiment of the present invention.
Fig. 6 is a flowchart for describing a flow of an information processing method in accordance with an embodiment of the present invention.
Fig. 7 is a diagram illustrating an example of a screen for selecting an assistance mode in accordance with an embodiment of the present invention.
Fig. 8 is a schematic view for describing a specific example of a crying stopping assistance mode in accordance with an embodiment of the present invention.
Fig. 9 is a flowchart for describing a detailed flow of a notification process in the crying stopping assistance mode illustrated in Fig. 6.
Fig. 10 is a flowchart for describing a detailed flow of a notification process in a get-to-sleep assistance mode illustrated in Fig. 6.
Fig. 11 is a schematic view for describing a specific example of the get-to-sleep assistance mode in accordance with an embodiment of the present invention.
Fig. 12 is a flowchart for describing a detailed flow of a notification process in an awakening prediction mode illustrated in Fig. 6.

### Description of Embodiments

### [Findings that form foundations for the present invention]

The inventors of the present invention have discovered a method for promoting stopping of crying of a crying infant and effectively laying the infant down to sleep. The method is a method in which: (procedure 1) holding a crying infant and walking with the infant is carried out without stopping the walking until the infant goes from a drowsy state into a sleep state (about 3 to 10 minutes, depending on interindividual differences and/or intraindividual differences varying depending on a previous state); and (procedure 2), after that, for a certain period of time (about 3 to 10 minutes, depending on interindividual differences and/or intraindividual differences varying depending on a previous state), holding the infant and sitting with the infant, which gives little stimulation, is carried out to induce the infant to go into a deeper sleep state, and, if the infant's eyes do not open, laying the infant down on a bed or the like is carried out. The respective times required for the procedures 1 and 2, which depend on interindividual differences and/or intraindividual differences varying depending on a previous state, can be optimized through prediction of the times with use of heart rate information and an infant state score.

The following will describe an experiment in which this finding was obtained and analysis results of the experiment. This experiment targeted 20 infants within 7 months of age. In this experiment, each caregiver performed childcare behaviors such as holding and walking (WalkHold), holding and sitting (SitHold), and laying down on a bed (COT) in accordance with experimenter's instructions. Further, while the caregiver was performing childcare behaviors, the infant's electrocardiogram and how the caregiver was performing the childcare behaviors were recorded with use of a Holter electrocardiograph and a video recorder. Further, on the basis of the records, a heart rate interval (hereinafter also referred to as inter beat interval (IBI)) and an infant state score (hereinafter also referred to as infant status score (ISS)) were calculated.

### (IBI: heart rate interval)

A value of the IBI increase as the state of an infant goes toward sleep. The IBI can be calculated on the basis of an electrocardiogram or the like, and it has been shown that similar results are obtained with use of pulse waves.

### (ISS: infant state score)

The ISS is a score that indicates the state of an infant from a crying state to a sleep state. In the analysis results, the ISS is a value obtained by calculation based on negative vocalization of an infant, a body motion thereof, opening and closing of eyes thereof, and the like. In this experiment, a theoretical value of the ISS is defined as a value that is in the range from -1 to 1 and that increases as the state of an infant goes toward sleep. Specifically, ISS=-1 indicates that an infant is in a 100% wailing state. The 100% wailing state means that the proportion of a time period of an infant's vocalization in a predetermined time period is 100%. Further, ISS=0 indicates that an infant has stopped crying and is in an awake state. Further, ISS=1 indicates that an infant is in a deep sleep state. For example, if the ISS exceeds 0, an infant is in a drowsy to deep sleep state. Further, if the ISS is more than -0.5 and less than 0, an infant is in a fussy state. Further, if the ISS is not more than -0.5, an infant is in an intensely crying state.

### (Analysis result 1)

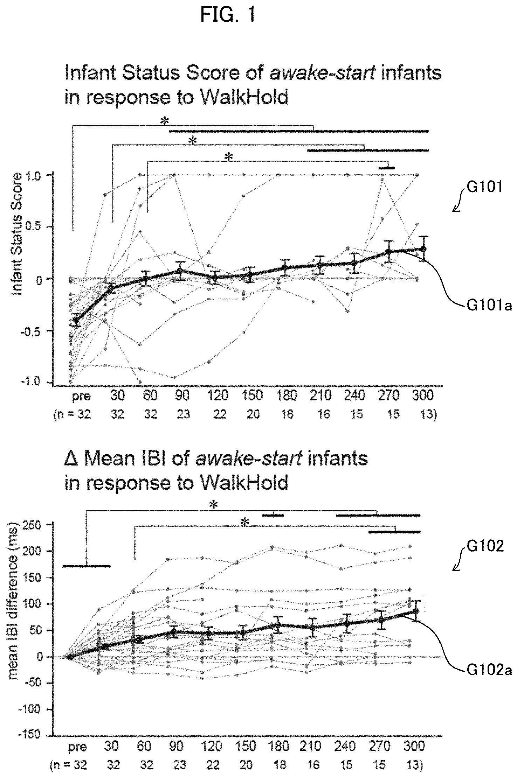

Analysis result 1 on holding and walking will be described with reference to Fig. 1. Fig. 1 is a graph for describing the analysis result 1 on holding and walking. As shown in Fig. 1, in a graph G101, the horizontal axis represents the length of time elapsed since the start of holding and walking, and the vertical axis represents the ISS. Further, a polygonal line G101a represents a mean value of the ISS of 18 infants (32 measurements). Further, in a graph G102, the horizontal axis represents the length of time elapsed since the start of holding and walking, and the vertical axis represents a change in the IBI from the start (ΔIBI). Further, a polygonal line G102a represents a mean value of the ΔIBI of 18 infants (32 measurements). From these graphs and data that are not included in the present specification, it is clear that, when a caregiver holds and walks with a crying infant, the infant effectively stops crying within 30 seconds (the ISS gets close to 0), and ΔIBI is significantly increased. Further, it is clear that, when the caregiver further keeps holding and walking without stopping, the infant goes into a drowsy to sleep state (the ΔIBI and the ISS increase) after a lapse of about 300 seconds since the start of holding and walking.

### (Analysis result 2)

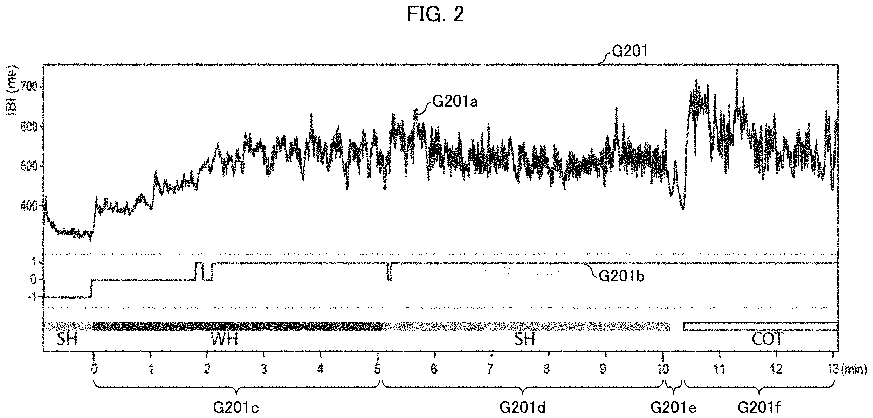

The analysis result 2 on a time period during which, after the caregiver has held and walked with the crying infant, and the infant has fallen asleep, the caregiver holds and sits with the infant and then lays the infant down to sleep will be described with reference to Fig. 2. Fig. 2 is a graph for describing the analysis result 2 on the time period during which, after the caregiver has held and walked with the crying infant, the caregiver holds and sits with the infant and then lays the infant down to sleep. Here, as shown in Fig. 2, a graph G201 is a graph of the progression of the IBI from the start of holding and sitting with the infant after the caregiver has held and walked with the infant, and the infant has fallen asleep. The horizontal axis represents an elapsed time. Further, the vertical axis represents the IBI. Further, a polygonal line G201a represents the IBI of a certain infant. A polygonal line G201b represents the ISS. A time period G201c is a time period of holding and walking. A time period G201d is a time period of holding and sitting. A time period G201e is a time period from a time at which the caregiver stands up while holding the infant and walks to the bed to a time at which the caregiver places the infant on the bed. A time period G201f is a time period during which the infant is moved away from the caregiver and is lying on the bed. As is clear from this graph, the IBI of the infant gradually increases in the time period G201c of holding and walking. Further, the ISS changes to 1, which indicates that the infant has fallen asleep. In addition, the IBI of the infant temporarily decreases in the time period G201e before the infant is placed on the bed, as compared with the time period G201d of holding and sitting. However, after that, the IBI is recovered in the time period G201f during which the infant is lying on the bed, and the IBI is much higher than the IBI in the period G201d of holding and sitting. That is, it is clear that, for the infant in a drowsy-to-sleep state, laying the infant down on the bed makes the IBI significantly higher and brings a deeper sleep than holding and sitting.

### (Analysis result 3)

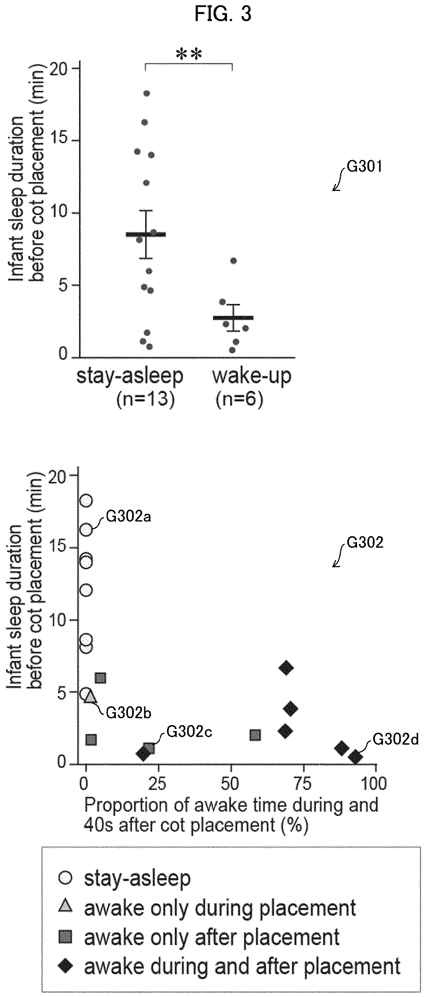

Analysis result 3 on the length of time spent on holding and sitting will be described with reference to Fig. 3. Fig. 3 is a graph for describing the analysis result 3 on the length of time spent on holding and sitting. As shown in Fig. 3, in a graph G301, the vertical axis represents the length of time elapsed before the caregiver laid the infant down on the bed after the infant had fallen asleep. As shown by the horizontal axis, the infants were divided into infants who woke up within 20 seconds after having been laid down on the bed (wake-up) and infants who did not wake up within 20 seconds after having been laid down on the bed (stay-asleep). As is clear from this graph, in the case of the infant who was placed on the bed and woke up again, the length of time elapsed before the caregiver laid the infant down on the bed after the infant had fallen asleep was short, as compared to the infant who stayed asleep even after the infant had been placed on the bed. In the graph G302, the vertical axis represents the length of the time elapsed before the caregiver laid the infant down on the bed after the infant had fallen asleep. The horizontal axis represents the proportion of the length of time during which the infant was awake in a time period from a time at which the caregiver started placing the infant on the bed to a time at which 40 seconds elapsed since the placement on the bed. A circular marker G302a indicates data on the infant who was sleeping all the time until 40 seconds elapsed after the placement on the bed since the start of placement on the bed. A triangular marker G302b indicates data on the infant who got awake in the middle of being placed on the bed, but was sleeping after having been placed on the bed. A quadrangular marker G302c indicates data on the infant who was sleeping in the middle of being placed on the bed, but got awake after having been placed on the bed. A rhombic marker G302d indicates data on the infant who got awake in the middle of being placed on the bed and remained awake even after the placement on the bed. Thus, it was found that, in a case where the length of time elapsed before the caregiver lays the infant down on the bed since the infant has fallen asleep is not more than 5 minutes, many infants wake up again in the middle of being placed on the bed or after having been placed on the bed. In contrast, in a case where the length of time elapsed before the caregiver lays the infant down on the bed since the infant has fallen asleep is not less than 7 minutes, all the infants remain in a sleep state. That is, it is clear that laying the infant down on the bed after 5 to 7 minutes have elapsed since the infant fell asleep can decrease the probability that, after having been laid down, the infant wakes up again and then resumes crying.

The inventors have derived the above-described procedure 1 for promoting stopping of crying of an infant from the analysis result 1. Further, the inventors have derived the procedure 2 for promoting sleep of an infant from the analysis results 2 and 3. In addition, the inventors have derived a method of carrying out the procedure 1 and then carrying out the procedure 2 as a method for getting a crying infant to sleep. The following will describe an embodiment of the present invention that assists childcare behaviors on the basis of these findings.

### [Embodiment]

An embodiment of the present invention will be described in detail.

### (Hardware configuration of information processing system 1)

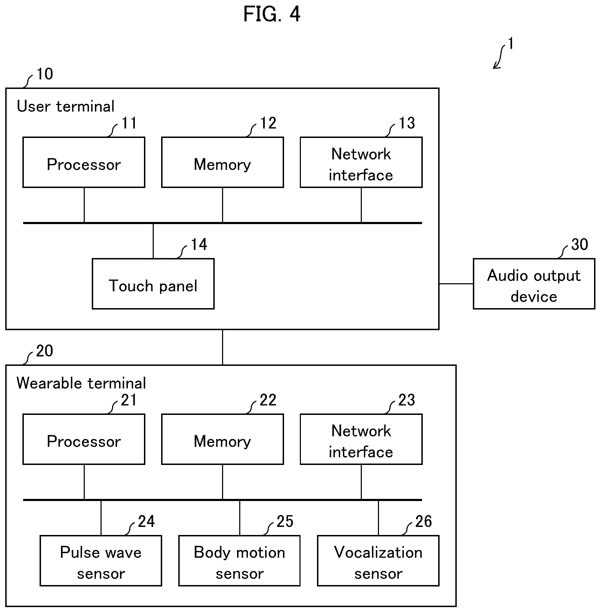

A configuration of an information processing system 1 in accordance with an embodiment of the present invention will be described with reference to Fig. 4. Fig. 4 is a block diagram illustrating an example of a hardware configuration of the information processing system 1. As illustrated in Fig. 4, the information processing system 1 includes a user terminal 10, a wearable terminal 20, and an audio output device 30. The user terminal 10 is communicably connected to the audio output device 30 and to the wearable terminal 20. In the present embodiment, an example in which these terminals are capable of wireless communications between the terminals will be described.

The user terminal 10 is a terminal that is used by a user U who performs a childcare behavior with respect to an infant T. The user terminal 10 is constituted by, for example, a smartphone. However, a computer constituting the user terminal 10 is not limited to these. The user terminal 10 includes a processor 11, a memory 12, a network interface 13, and a touch panel 14. The processor 11 reads and executes a program stored in the memory 12 to execute at least a part of an information processing method S1 described later. The memory 12 stores a program for causing the processor 11 to execute at least a part of the information processing method S1. The network interface 13 connects to a network for carrying out wireless communications with the wearable terminal 20 and the audio output device 30. The network interface 13 is an interface that connects to, for example, a wireless local area network (LAN) or a short-range wireless communication network. Specific examples of the short-range wireless communication network include near field communication (NFC), Bluetooth (registered trademark), and the like. However, the network to which the network interface 13 connects is not limited to those described above. The touch panel 14 displays information output by the processor 11. In addition, the touch panel 14 receives an operation performed by the user U. The operation performed by the user U includes, for example, an operation of touching the touch panel 14 with a finger, a touch pen, or the like.

The wearable terminal 20 is constituted by, for example, a small computer that is wearable on the infant T. The part of the body on which the wearable terminal 20 is worn is, for example, an ankle, a calf, a foot dorsum, or the like. A member with which the wearable terminal 20 is worn is, for example, an elastic band (not illustrated). However, the part of the body on which the wearable terminal 20 is worn and the member with which the wearable terminal 20 is worn are not limited to those described above. The wearable terminal 20 includes a processor 21, a memory 22, a network interface 23, a pulse wave sensor 24, a body motion sensor 25, and a vocalization sensor 26. The processor 21 reads and executes a program stored in the memory 22 to execute at least a part of an information processing method S1 described later. The memory 22 stores a program for causing the processor 21 to execute at least a part of the information processing method S1. The network interface 23 connects to a network for carrying out wireless communications with the user terminal 10. Specific examples of the network interface 23 are the same as the specific examples of the network interface 13 described above.

The pulse wave sensor 24 is a sensor that detects a pulse wave of the infant T. For example, the pulse wave sensor 24 is an optical pulse wave sensor, but is not limited thereto. The body motion sensor 25 is a sensor that detects a body motion of the infant T. For example, the body motion sensor 25 is an acceleration sensor, a gyro sensor, or the like, but is not limited thereto. The vocalization sensor 26 is a sensor that detects vocalization of the infant T. The vocalization sensor 26 is a microphone or the like, but is not limited thereto. The pulse wave sensor 24, the body motion sensor 25, and the vocalization sensor 26 are examples of a "sensor that detects the state of an infant" recited in the claims, and are examples of a "sensor that detects at least one selected from the group consisting of a pulse wave, a body motion, and vocalization". Further, information indicating the pulse wave, the body motion, and the vocalization acquired from these sensors is an example of the "sensor information" recited in the claims.

The audio output device 30 outputs an audio output from the user terminal 10. For example, the audio output device 30 is constituted by a headphone, an earphone, or the like that are wearable on ears of the user U, but is not limited thereto.

### (Functional configuration of information processing system 1)

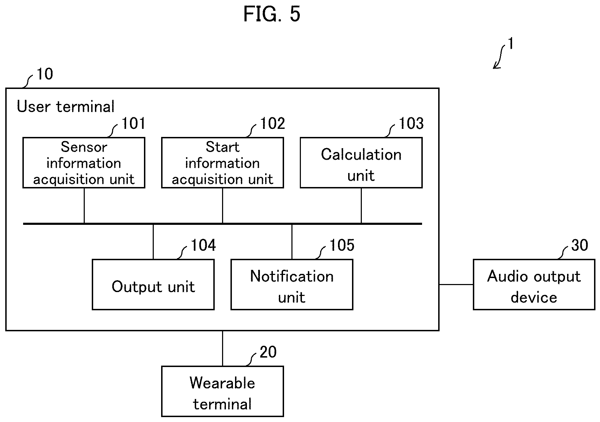

A functional configuration of the information processing system 1 will be described with reference to Fig. 5. Fig. 5 is a block diagram illustrating an example of a functional configuration of the information processing system 1. As illustrated in Fig. 5, in the information processing system 1, the user terminal 10 includes a sensor information acquisition unit 101, a start information acquisition unit 102, a calculation unit 103, an output unit 104, and a notification unit 105. The functional blocks are implemented by the above-described processor 11 executing the above-described program stored in the memory 12.

Note that, in the present embodiment, these functional blocks are disposed in the user terminal 10. However, one or some or all of these functional blocks may be disposed in the wearable terminal 20. One or some or all of these functional blocks may be disposed in a server (e.g., a server on a cloud) (not illustrated). In such a case, each functional block disposed in the user terminal 10, the wearable terminal 20, or the server realizes the function by transmitting and receiving necessary information to and from each other.

The sensor information acquisition unit 101 acquires sensor information from a sensor that detects the state of the infant T. The start information acquisition unit 102 acquires information indicating that the user U has started holding the infant T and walking with the infant T. On the basis of the sensor information, the calculation unit 103 calculates at least one selected from the group consisting of heart rate information related to a heart rate of the infant T and an infant state score indicating any one of states of the infant T from a wailing state to a sleep state. The output unit 104 outputs, in real time, information indicating at least one selected from the group consisting of the heart rate information and the infant state score. The notification unit 105 outputs notification information for notifying the user U of a childcare behavior to be performed to promote stopping of crying of the infant T or sleep of the infant T on the basis of at least one selected from the group consisting of the length of time elapsed since the user U has started holding and walking with the infant T, the heart rate information, and the infant state score. Details of these units will be described in "Flow of information processing method S1" below.

### (Flow of information processing method S1)

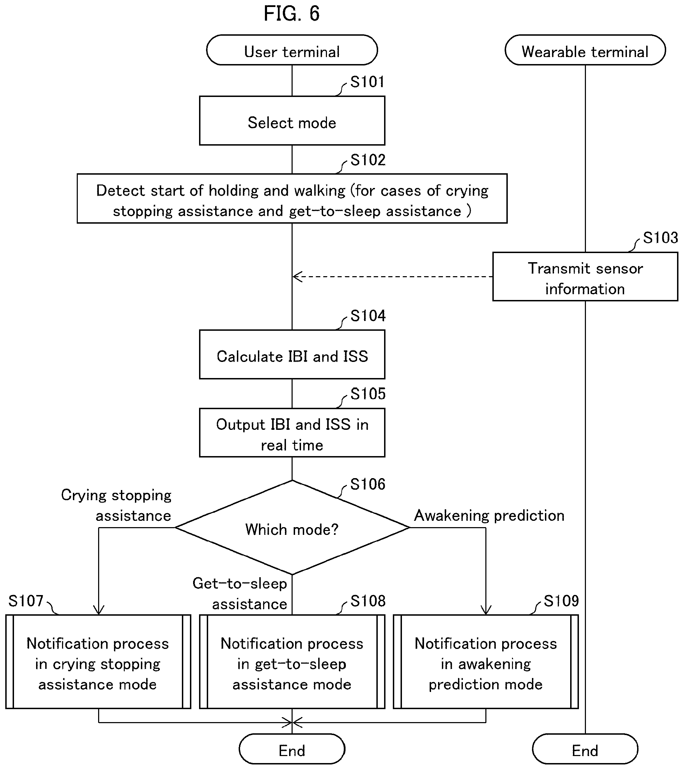

A flow of the information processing method S1 that is carried out by the information processing system 1 configured as described above will be described with reference to Fig. 6. Fig. 6 is a flowchart for describing the flow of the information processing method S1. As illustrated in Fig. 6, the information processing method S1 includes steps S101 to S109.

In step S101, the processor 11 acquires information indicating an assistance mode that has been selected by an operation of the user U. The assistance mode is a mode for assisting a childcare behavior of the user U with respect to the infant T. Here, the assistance mode includes a crying stopping assistance mode, a get-to-sleep assistance mode, and an awakening prediction mode.

The crying stopping assistance mode is a mode for promoting stopping of crying of the infant T who is crying. This assistance mode assists a childcare behavior of the user U, for example, in a case where the infant T is crying in a situation where it does not matter that the infant T is awake if the infant T is stopping crying (for example, during the daytime or the like). The childcare behavior to be performed to promote stopping of crying of the infant T includes a behavior related to holding and walking. Details of this assistance mode will be described later.

The get-to-sleep assistance mode is a mode for promoting sleep of the infant T who is crying. This assistance mode assists a childcare behavior of the user U, for example, in a case where the infant T is crying in a situation where it is desirable that the infant T sleeps (for example, during a nap time, during the nighttime, or the like). The childcare behavior to be performed to promote getting the infant T to sleep includes behaviors related to: holding and walking; holding and sitting; and laying down to sleep. Details of this assistance mode will be described later.

The awakening prediction mode is a mode for predicting awakening of the infant T who is sleeping. This assistance mode assists a childcare behavior of the user U before and after awakening of the infant T by, for example, predicting awakening of the infant T who is sleeping. Details of this assistance mode will be described later.

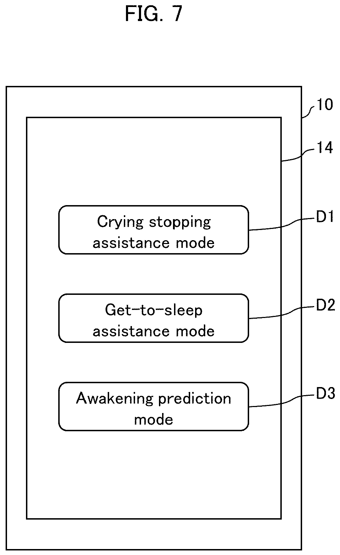

A specific example of step S101 will be described with reference to Fig. 7. Fig. 7 is a diagram illustrating an example of a screen for selecting an assistance mode. As illustrated in Fig. 7, the processor 11 displays, on the touch panel 14, user interface (hereinafter referred to as UI) objects D1 to D3. Each of the UI objects D1 to D3 is an object that receives an operation for selecting an assistance mode. Each of the UI objects D1 to D3 corresponds to a corresponding one of the assistance modes (the crying stopping assistance mode, the get-to-sleep assistance mode, and the awakening prediction mode) indicated in each of the objects. The processor 11 acquires information indicating the assistance mode corresponding to an object for which an operation that has been received.

In step S102 (start information acquisition step) in Fig. 6, the processor 11 (start information acquisition unit 102) acquires information indicating that the user U has started holding the infant T and walking with the infant T. Note that this step is carried out in the case of the crying stopping assistance mode or the get-to-sleep assistance mode, and is omitted in the case of the awakening prediction mode.

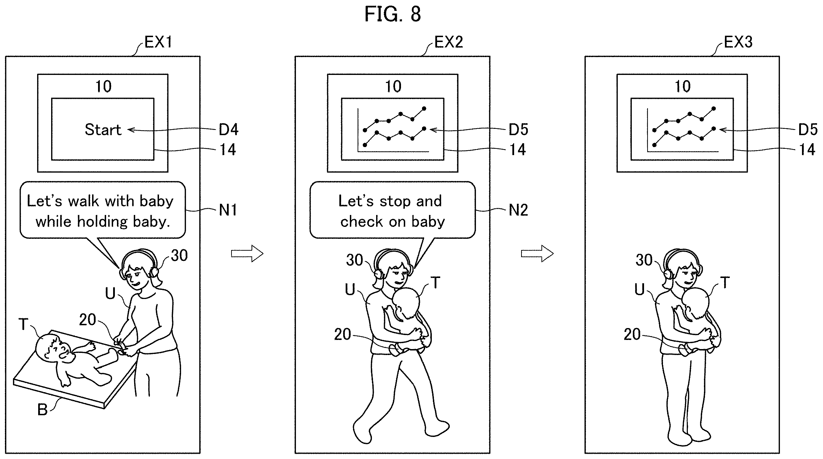

A specific example of step S102 will be described with reference to Fig. 8. Fig. 8 is a schematic view for describing a specific example of the crying stopping assistance mode. As illustrated in a schematic view EX1 in Fig. 8, the processor 11 displays a UI object D4 on the touch panel 14. The UI object D4 is an object that receives an operation for starting the assistance mode. The user U has an infant T who is crying in a bed B wear the wearable terminal 20 and operates the UI object D4. Further, upon receiving the operation performed on the UI object D4, the processor 11 outputs notification information N1 to the audio output device 30. The notification information N1 is information for providing a notification for prompting the user U to start holding the infant T and walking with the infant T, and is, for example, audio information saying "Let's walk with the baby while holding the baby". However, details of the notification information N1 and an output destination thereof are not limited to the examples described above. Thus, as illustrated in a schematic view EX2, the user U starts holding the infant T and walking with the infant T. As described above, in this specific example, when receiving the operation performed on the UI object D4, the processor 11 acquires information indicating the start of holding and walking.

In step S103 in Fig. 6, the processor 21 of the wearable terminal 20 acquires sensor information (information indicating a pulse wave, a body motion, and a voice) that has been detected by the pulse wave sensor 24, the body motion sensor 25, and the vocalization sensor 26. Further, the processor 21 transmits the acquired sensor information to the user terminal 10. The processor 21 repeats transmission of the sensor information to the user terminal 10. The processor 21 may transmit the sensor information in response to a request from the user terminal 10. The processor 21 may transmit the sensor information to the user terminal 10 at a predetermined interval.

In step S104 (sensor information acquisition step and calculation step), the processor 11 (sensor information acquisition unit 101) of the user terminal 10 acquires the sensor information from the wearable terminal 20. Further, the processor 11 (calculation unit 103) calculates at least one selected from the group consisting of the IBI and the ISS on the basis of the acquired sensor information. The IBI is an example of the "heart rate information related to a heart rate of the infant T" recited in the claims. The ISS is an example of an "infant state score indicating any one of states of the infant T from a wailing state to a sleep state" recited in the claims. The processor 11 repeats calculation of at least one selected from the group consisting of the IBI and the ISS.

### (Seven stages of ISS)

Here, in the initial setting, the processor 11 calibrates (adjusts) a relationship between the ISS and the IBI and the sensor information so as to represent individual characteristics of a target infant T. Details of the initial setting will be described later. In the present embodiment, in place of the above-described theoretical values of the ISS in the range from -1 to 1, seven stages of the ISS obtained by the calibration are used. Specifically, the processor 11 associates values of seven stages of the ISS with, for example, the IBI, the sensor information, and the like.

Specific examples of the seven stages are presented below.
- Stage 1: 100% to 51% wailing (the proportion of a vocalization time of the infant T in a unit time is 100% to 51%).
- Stage 2: 1% to 50% wailing (the above-described proportion is 1% to 50%)
- Stage 3: actively awake state without wailing (more body motions than those in Stage 4)
- Stage 4: inactively awake state without wailing (fewer body motions than those in Stage 3)
- Stage 5: drowsy state (looks sleepy, yawns, opens and closes eyes)
- Stage 6: light sleep (eyes are closed, but there is sometimes a movement of a skin around the eyes or a body motion)
- Stage 7: deep sleep (eyes are closed, and hands and a face do not move)

However, specific examples of the seven stages are not limited to those described above. For example, the wailing ranges that define Stage 1 and Stage 2 are not limited to the above-described ranges, and may be other ranges. For example, the value of 50% (51%) wailing which is a boundary between Stage 1 and Stage 2 may be other value. Further, a lower limit of the wailing range that defines Stage 2 may be a value greater than 1%. In addition, the range or threshold value defining each stage may be changeable.

In step S105 (an output process, an output step), the processor 11 (output unit 104) outputs, in real time, information indicating at least one selected from the group consisting of the IBI and the ISS. Thereafter, the processor 11 continues the process of outputting the IBI and the ISS in real time. The output destination is, for example, the touch panel 14 and/or the audio output device 30.

A specific example of step S105 will be described with reference to Fig. 8. As illustrated in the schematic view EX2 in Fig. 8, the processor 11 outputs, to the touch panel 14, change-over-time information D5 indicating a change over time in the IBI and a change over time in the ISS. In this example, the change-over-time information D5 is a graph in which the horizontal axis represents a time axis, and the vertical axis represents the IBI and the ISS. Further, the processor 11 updates the change-over-time information D5 in real time.

In step S106 in Fig. 6, the processor 11 determines which of the assistance modes is selected, and branches the subsequent processing according to the result of the determination.

Step S107 (a notification process, a notification step) is carried out in a case where the crying stopping assistance mode is selected. In step S107, the processor 11 (notification unit 105) carries out a notification process in the crying stopping assistance mode. Specifically, the processor 11 outputs notification information for notifying the user U of a childcare behavior to be performed to promote stopping of crying of the infant T on the basis of at least one selected from the group consisting of the length of time elapsed since the user U has started holding and walking with the infant T, the IBI, and the ISS. The notification information includes first notification information, second notification information, and fourth notification information, which will be described later. Details of this step will be described later with reference to a different drawing.

Step S108 (a notification process, a notification step) is carried out in a case where the get-to-sleep assistance mode is selected. In step S108, the processor 11 (notification unit 105) carries out a notification process in the get-to-sleep assistance mode. Specifically, the processor 11 outputs notification information for notifying the user U of a childcare behavior to be performed to promote sleep of the infant T on the basis of at least one selected from the group consisting of the length of time elapsed since the user U has started holding and walking with the infant T, the IBI, and the ISS. The notification information includes first notification information, second notification information, third notification information, and fourth notification information, which will be described later. Details of this step will be described later with reference to a different drawing.

Step S109 (a notification process, a notification step) is carried out in a case where the awakening prediction mode is selected. In step S109, the processor 11 (notification unit 105) carries out a notification process in the awakening prediction assistance mode. Details of this step will be described later with reference to a different drawing.

### (Flow of notification process in crying stopping assistance mode)

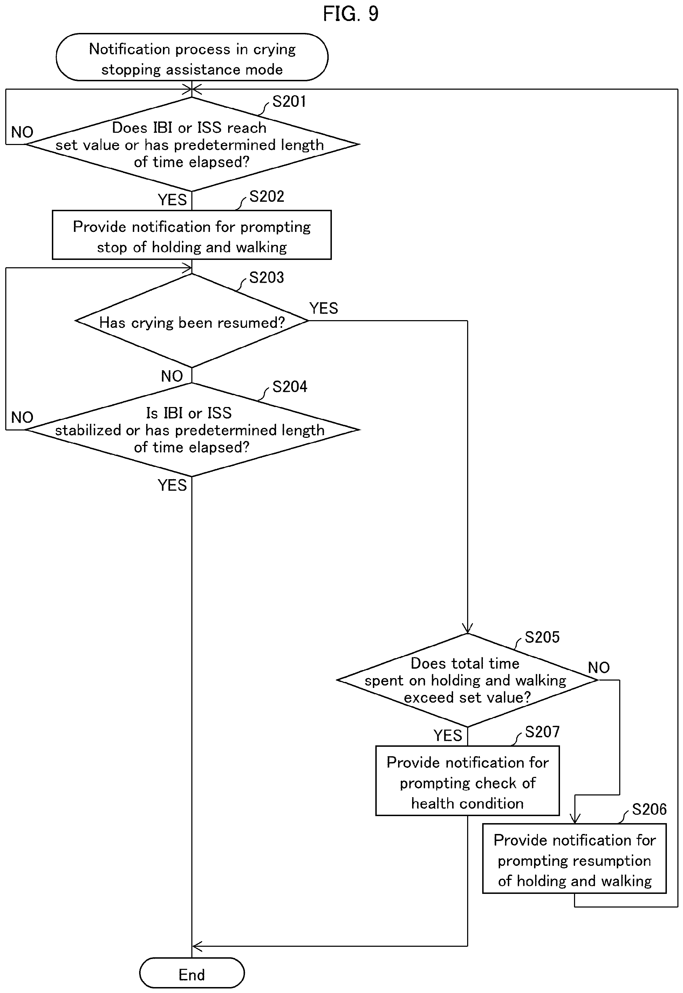

Details of step S107 (the notification process in the crying stopping assistance mode will be described with reference to Fig. 9. Fig. 9 is a flowchart for describing a detailed flow of step S107. As illustrated in Fig. 9, the process in step S107 includes steps S201 to S207.

In step S201, the processor 11 (notification unit 105) determines whether to output notification information N2 on the basis of at least one selected from the group consisting of the length of time elapsed since the detection of the start of holding and walking, the IBI, and the ISS. Details of the notification information N2 will be described later.

As a specific example, the processor 11 determines (i) whether the length of time elapsed since the detection of the start of holding and walking exceeds a predetermined length of time (threshold value T1), (ii) whether the IBI exceeds a set value (threshold value IBI1), and (iii) whether the ISS exceeds a set value (threshold value ISS1). In this case, the processor 11 may be configured to make a determination of "Yes" in a case where at least one of (i), (ii), and (iii) is satisfied and to make a determination of "No" in a case where any of (i), (ii), and (iii) is not satisfied. Alternatively, the processor 11 may be configured to make a determination of "Yes" in a case where all of (i), (ii), and (iii) are satisfied and to make a determination of "No" in a case where at least one of (i), (ii), and (iii) is not satisfied.

As the threshold value T1, the threshold value IBI1, and the threshold value ISS1, initially set values are used. Details of the initial setting will be described later.

In a case where the determination of "No" is made in step S201, the processor 11 repeats this step until the processor 11 makes a determination of "Yes". Thus, the user U continues holding the infant T and walking with the infant T while checking changes over time in the IBI and the ISS which are displayed on the touch panel 14.

In a case where the determination of "Yes" is made in step S201, step S202 is carried out. In step S202, the processor 11 outputs the notification information N2. The notification information N2 is information for providing a notification for prompting the user U to stop holding and walking, and is an example of the first notification information recited in the claims. Note that the notification information N2 may further include providing a notification for prompting to check on the infant T. In addition, the notification information N2 may further include providing a notification for prompting to check the IBI and the ISS which are displayed on the touch panel 14.

Thus, the notification information output by the processor 11 in the information processing method S1 includes the notification information N2 (first notification information). Further, the processor 11 outputs the notification information N2 (first notification information) on the basis of at least one selected from the group consisting of the length of time elapsed since the user U has started holding and walking, the IBI, and the ISS (a result of the determination on (i), (ii), and (iii) in step S201 is "Yes") (step S202).

A specific example of step S202 will be described with reference to Fig. 8. As illustrated in the schematic view EX2 in Fig. 8, the processor 11 outputs, to the audio output device 30, audio information saying "Let's stop and check on the baby" as the notification information N2. However, details of the notification information N2 and an output destination thereof are not limited to the examples described above. Thus, as illustrated in a schematic view EX3, the user U stops while holding the infant T and then checks on the infant T.

In step S203 in Fig. 9, the processor 11 determines whether crying of the infant T has been resumed. For example, the processor 11 can determine whether crying has been resumed on the basis of the sensor information acquired from the vocalization sensor 26. For example, the processor 11 may determine that crying has been resumed in a case where the proportion of a vocalization time of the infant T in a unit time is equal to or greater than a threshold value SEN1. Here, in the present embodiment, as the threshold value SEN1, an initially set value is used. Details of the initial setting will be described later.

In a case where the determination of "Yes" is made in step S203, step S205 described later is carried out. In a case where the determination of "No" is made in step S203, step S204 that follows step S203 is carried out.

In step S204, the processor 11 determines whether the infant is in a calm state on the basis of at least one selected from the group consisting of the length of time elapsed since the output of the notification information N2, the IBI, and the ISS.

As a specific example, the processor 11 determines (i) whether the length of time elapsed since the output of the notification information N2 (in other words, since the stop of holding and walking) exceeds a predetermined length of time (threshold value T2), (ii) whether the IBI is stabilized, and (iii) whether the ISS is stabilized. In this case, the processor 11 may be configured to make a determination of "Yes" in a case where at least one of (i), (ii), and (iii) is satisfied and to make a determination of "No" in a case where any of (i), (ii), and (iii) is not satisfied. Alternatively, the processor 11 may be configured to make a determination of "Yes" in a case where all of (i), (ii), and (iii) are satisfied and to make a determination of "No" in a case where at least one of (i), (ii), and (iii) is not satisfied.

Note that, as the threshold value T2, an initially set value is used. Details of the initial setting will be described later. In addition, "the IBI (or the ISS) is stabilized" may be, for example, such that "the IBI (or the ISS) during the latest predetermined period is not decreased as compared to a point in time when the notification information N2 has been output and varies within a predetermined range".

In a case where the determination of "No" is made in step S204, the processor 11 repeats the processes in step S203 and the following step. Thus, as illustrated in the schematic view EX3, the user U checks on the infant T while stopping and holding the infant T.

In a case where the determination of "Yes" is made in step S204, the infant T is in a state of stopping crying and being calm. Therefore, in this case, the procedure in the crying stopping assistance mode is ended.

In a case where the determination of "Yes" is made in step S203, it means that crying of the infant T is resumed. In this case, in step S 205, the processor 11 determines whether a total time spent on holding and walking exceeds a threshold value T3. In a case where the determination of "Yes" is made in this step, step S207 described later is carried out. In a case where the determination of "No" is made in this step, step S206 that follows this step is carried out. Here, as the threshold value T3, an initially set value is used. Details of the initial setting will be described later.

In step S206, the processor 11 outputs notification information N3 to the audio output device 30. The notification information N3 is information for providing a notification for prompting the user U to resume holding and walking. Further, after having carried out step S206, the processor 11 repeats the processes in step S201 and the following steps. Thus, in the specific example in Fig. 8, the user U returns to the schematic view EX2 and repeats the resumption of holding and walking and the stopping of holding and walking.

Further, in a case where the determination of "Yes" is made in step S205, it means that the total time spent on holding and walking exceeds the threshold value T3. In this case, step S207 that follows step S205 is carried out.

In step S207, the processor 11 outputs notification information N4 to the audio output device 30. The notification information N4 is information for providing a notification for prompting the user U to check a health condition of the infant T, and is an example of the fourth notification information recited in the claims. In other words, the notification information output by the processor 11 in the information processing method S1 includes the notification information N4 (fourth notification information). Further, in a case where the total time spent on holding and walking exceeds the threshold value T3 ("Yes" in step S205), the processor 11 outputs the notification information N4 (fourth notification information) (step S207). This allows the user U to stop the use of the crying stopping assistance mode and pay attention to the health condition of the infant T.

Thus, in the crying stopping assistance mode, the user U starts and stops holding and walking in accordance with the output of the notification information N1 to the notification information N4, so that it is possible to efficiently promote stopping of crying of the infant T who is crying.

### (Flow of notification process in get-to-sleep assistance mode)

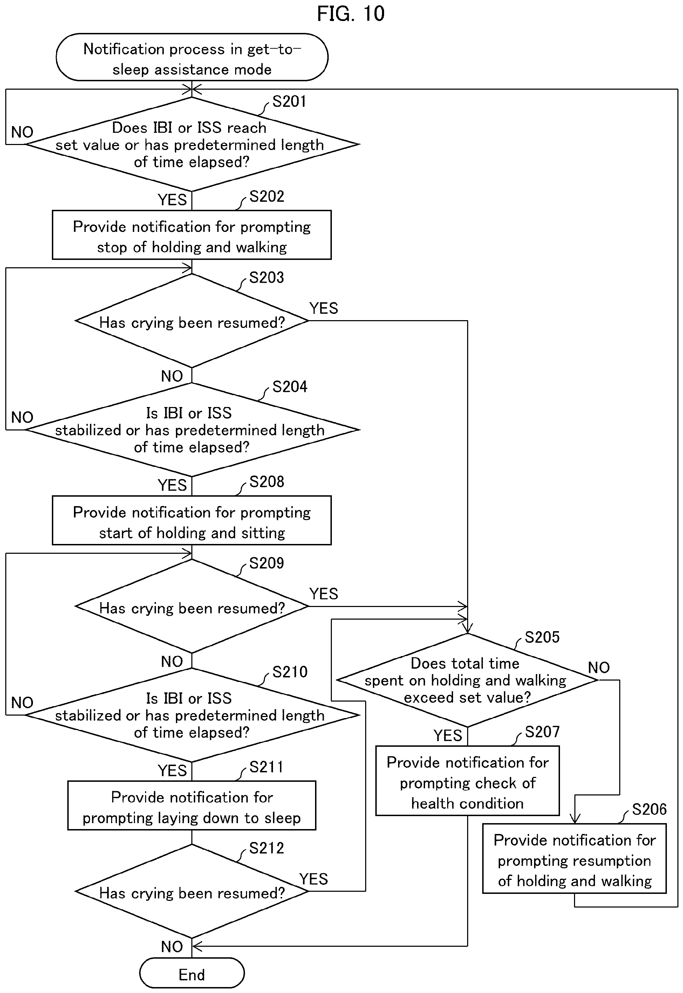

Details of step S108 will be described with reference to Fig. 10. Fig. 10 is a flowchart for describing a detailed flow of step S108 (notification process in the get-to-sleep assistance mode). As illustrated in Fig. 10, the process in step S108 includes steps S201 to S212. Steps S201 to S207 are as described with reference to Fig. 9 in the procedure in the crying stopping assistance mode. However, in the determination in step S201 in the get-to-sleep assistance mode, a threshold value IBI2, which differs from the threshold value IBI1 in the crying stopping assistance mode, and a threshold ISS2, which differs from the threshold value ISS1, are used. Further, in the determination in step S203 in the get-to-sleep assistance mode, a threshold value SEN2, which differs from the threshold value SEN1 in the crying stopping assistance mode, is used. Here, steps S208 to S212 will be described.

Step S208 is carried out in a case where the determination of "Yes" is made in step S204. In this case, the infant T is in a calm state while being held by the user U who is stopping. Therefore, in step S208, the processor 11 (notification unit 105) outputs notification information N5. The notification information N5 is information for providing a notification for prompting the user U to start holding and sitting, and is an example of the second notification information recited in the claims. In other words, the notification information output by the processor 11 in the information processing method S1 includes the notification information N5 (second notification information). Further, the processor 11 outputs the notification information N5 (second notification information) on the basis of at least one selected from the group consisting of the length of time elapsed since the output of the notification information N2 (first notification information), the IBI, and the ISS (a result of the determination on (i), (ii), and (iii) in step S204 is Yes) (step S208).

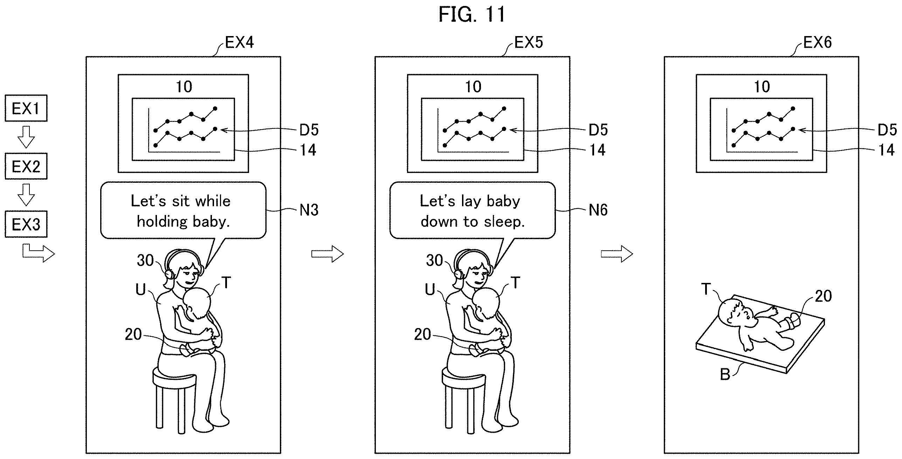

A specific example of step S208 will be described with reference to Fig. 11. Fig. 11 is a schematic view for describing a specific example of the get-to-sleep assistance mode. In the specific example in Fig. 11, figures EX1 to EX3 represent schematic views similar to the schematic views EX1 to EX3 illustrated in Fig. 8. A schematic view EX4 in Fig. 11 is a schematic view that follows the schematic view EX3. As illustrated in the schematic view EX4 in Fig. 11, the processor 11 outputs, to the audio output device 30, audio information saying "Let's sit while holding the baby". However, details of the notification information N5 and an output destination thereof are not limited to the examples described above. Thus, the user U starts holding the infant T and sitting with the infant T.

In step S209 in Fig. 10, the processor 11 determines whether crying of the infant T has been resumed. Details of this step are the same as those of step S203.

In a case where the determination of "No" is made in step S209, step S210 that follows step S209 is carried out. In this case, in a state in which the user U is holding the infant T and sitting with the infant T, crying is not resumed. In step S210, the processor 11 determines whether to output notification information N6 (third notification information) on the basis of at least one selected from the group consisting of the length of time elapsed since the output of the notification information N5, the IBI, and the ISS. Details of the notification information N6 will be described later.

As a specific example, the processor 11 determines (i) whether the length of time elapsed since the output of the notification information N5 (in other words, since the start of holding and sitting) exceeds a predetermined length of time (threshold value T4), (ii) whether the IBI is stabilized, and (iii) whether the ISS is stabilized. In this case, the processor 11 may be configured to make a determination of "Yes" in a case where at least one of (i), (ii), and (iii) is satisfied and to make a determination of "No" in a case where any of (i), (ii), and (iii) is not satisfied. Alternatively, the processor 11 may be configured to make a determination of "Yes" in a case where all of (i), (ii), and (iii) are satisfied and to make a determination of "No" in a case where at least one of (i), (ii), and (iii) is not satisfied.

Note that, as the threshold value T4, an initially set value is used. Details of the initial setting will be described later. Further, "the IBI (or the ISS) is stabilized" is as described in step S204. However, the point in time when the notification information N5 has been output is used as the point in time for comparison of whether the IBI (or the ISS) is stabilized.

In a case where the determination of "No" is made in step S210, the processor 11 repeats the processes in step S206 and the following step. Thus, the user U continues holding the infant T and sitting with the infant T.

In a case where the determination of "Yes" is made in step S210, step S211 that follows step S210 is carried out. In step S211, the processor 11 outputs the notification information N6. The notification information N6 is information for providing a notification for prompting the user U to lay down to sleep, and is an example of the third notification information recited in the claims. In other words, the notification information output by the processor 11 in the information processing method S1 includes the notification information N6 (third notification information). Further, the processor 11 outputs the notification information N6 (third notification information) on the basis of at least one selected from the group consisting of the length of time elapsed since the output of the notification information N5 (second notification information), the IBI, and the ISS (a result of the determination on (i), (ii), and (iii) in step S210 is "Yes") (step S211).

A specific example of step S211 will be described with reference to Fig. 11. As illustrated in the schematic view EX5, the processor 11 outputs, to the audio output device 30, audio information saying "Let's lay the baby down to sleep". However, details of the notification information N6 and an output destination thereof are not limited to the examples described above. Thus, as illustrated in a schematic view EX6, the user U moves the infant T away from the body of the user U and places the infant T on the bed B, in order to lay the infant T down to sleep.

In step S212 in Fig. 10, the processor 11 determines whether crying of the infant T has been resumed. Details of this step are the same as those of step S203.

In a case where the determination of "No" is made in step S212, it means that crying of the infant T is not resumed, and thus the infant T is sleeping and is in a preferable state. Therefore, in this case, the procedure in the get-to-sleep assistance mode is ended.

In a case where the determination of "Yes" is made in step S212, it means that crying of the infant T who has been laid down to sleep is resumed. Further, in a case where the determination of "Yes" is made in step S209, it means that crying of the infant T with whom the user U has held and sat is resumed. In this case, steps S205 to S207 described above are carried out in the same manner as in the case where crying has been resumed in the crying stopping assistance mode. Thus, the user U returns to the schematic view EX2 and repeats resuming holding and walking, stopping holding and walking, starting holding and sitting, laying down to sleep, and the like. Further, in a case where the total time spent on holding and walking exceeds the threshold value T3, the user U can stop the use of the get-to-sleep assistance mode and pay attention to the health condition of the infant T.

Thus, in the get-to-sleep assistance mode, the user U starts and stops holding and walking, performs holding and sitting, and lays down to sleep in accordance with the output of the notification information N1 to the notification information N6, so that it is possible to efficiently promote sleep of the infant T who is crying.

### (Flow of notification process in awakening prediction mode)

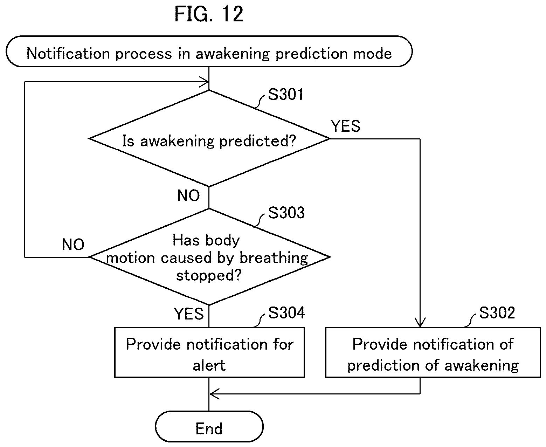

Details of step S109 (the notification process in the awakening prediction mode) will be described with reference to Fig. 12. Fig. 12 is a flowchart for describing a detailed flow of step S109. As illustrated in Fig. 12, the process in step S109 includes steps S301 to S304.

In step S301, the processor 11 (notification unit 105) determines whether awakening of the infant T is predicted on the basis of at least one selected from the group consisting of a body motion and the IBI.

As a specific example, the processor 11 determines (i) whether the amount of body motion based on the sensor information acquired from the body motion sensor 25 exceeds a threshold value SEN3, and (ii) whether the IBI exceeds a threshold IBI3. In this case, the processor 11 may be configured to make a determination of "Yes" in a case where at least one of (i) and (ii) is satisfied and to make a determination of "No" in a case where any of (i) and (ii) is not satisfied. Alternatively, the processor 11 may be configured to make a determination of "Yes" in a case where all of (i) and (ii) are satisfied and to make a determination of "No" in a case where at least one of (i) and (ii) is not satisfied.

Note that, as the threshold value SEN3 and the threshold value IBI3, initially set values are used. Details of the initial setting will be described later.

In a case where the determination of "No" is made in step S301, step S303 described later is carried out. In a case where the determination of "Yes" is made in step S301, step S302 that follows step S301 is carried out.

In step S302, the processor 11 outputs notification information N7. The notification information N7 is information for providing, to the user U, a notification that awakening of the infant T is predicted. When the notification information N7 is output, the user U can predict the awakening and perform a childcare behavior even in a case where the user U stays at a place away from the lying infant T.

In a case where the determination of "No" is made in step S301, the processor 11 determines whether to alert that there is a possibility that breathing of the infant T has stopped. For example, the processor 11 performs the determination on the basis of (i) whether a body motion caused by breathing has stopped (whether the amount of body motion based on the sensor information acquired from the body motion sensor 25 falls below a threshold value SEN 4) and (ii) whether the IBI exceeds a threshold value IBI4. Note that the processor 11 may be configured to make a determination of "Yes" in a case where all of (i) and (ii) are "Yes" and to make a determination of "No" in a case where at least one of (i) and (ii) is "No". Further, the processor 11 may be configured to make a determination of "Yes" in a case where at least one of (i) and (ii) is "Yes" and to make a determination of "No" in a case where any of (i) and (ii) is "No". As the threshold value SEN4 and the threshold value IBI4, initially set values are used. Details of the initial setting will be described later.

In a case where the determination of "No" is made in step S203, the processor 11 repeats the processes in step S301 and the following step. In a case where the determination of "Yes" is made in step S303, step S304 that follows step S303 is carried out.

In step S304, the processor 11 outputs notification information N8. The notification information N8 is information for alerting that there is a possibility that breathing of the infant T has stopped. When the notification information N8 is output, the user U can know the possibility that breathing of the infant T has stopped even in a case where the user U stays at a place away from the lying infant T. Thus, it is possible to prevent sudden infant death syndrome (SIDS).

### [Initial setting]

The initial setting of the information processing system 1 will be described. The initial setting is performed at least once before the information processing method S1 is carried out for the infant T for the first time. In addition, after that, it is desirable that the initial setting be performed again at any point in time. For example, the initial setting may be performed about once a month in view of a change due to the growth of the infant T.

The initial setting includes steps S11 to S15 below. Note that at the point in time when the initial setting is performed, the wearable terminal 20 is in a state of being worn on the infant T.

In step S11, the processor 11 of the user terminal 10 establishes a connection with the wearable terminal 20 via the network interface 13. The establishment of the connection may be manually performed on the basis of an operation of the user U or may be automatically performed regardless of whether the operation of the user U is performed.

In step S12, the processor 11 acquires sensor information (pulse wave, body motion, and voice) in each state of the infant T (wailing state, awake state, and deep sleep state) twice or three times for 20 seconds each time, and stores the sensor information in association with each state. Note that the time period during which the sensor information is acquired is not limited to 20 seconds, and the number of times the sensor information is acquired is not limited to twice or three times. Here, the wailing state is a state in which the infant T is intensely crying. The awake state is a state in which the infant T is completely awake in a quiet state. The deep sleep state is a state in which the infant T is seemingly sleeping without moving.

For example, the processor 11 displays UI objects indicating the corresponding states on the touch panel 14. For example, when the infant T is in the wailing state, the user U operates a UI object corresponding to the wailing state. Upon receiving the operation, the processor 11 performs the acquisition of the sensor information for 20 seconds. After the lapse of 20 seconds, the processor 11 outputs information for inquiring the user U about "whether there was any change in the state of the infant T during recording". In addition, in a case where an input indicating that there was no change has been acquired, the processor 11 stores the sensor information in association with the wailing state.

In step S13, the processor 11 sets the above-described threshold values (T1 to T4, IBI1 to IBI4, ISS1 to ISS2, SEN1 to SEN4) on the basis of the sensor information which has been associated with each state.

In step S14, the processor 11 calibrates the relationship between each stage of the ISS and the IBI and the sensor information on the basis of the sensor information associated with each state.

In step S15, the processor 11 outputs the IBI and the ISS to the touch panel 14 in real time.

The following will describe a preferable example of the threshold values set by the initial setting. As described above, the threshold value T1, the threshold values IBI1 to IBI2, and the threshold values ISS1 to ISS2 are set to determine whether to provide a notification for prompting to stop holding and walking.

The threshold value T1 is a value of a preferable length of time elapsed before holding and walking is stopped (the length of time elapsed since the start of holding and walking). For example, it is desirable that the threshold value T1 be set to 5 minutes on the basis of the "Findings that form foundations for the present invention" described above. Note that it is desirable that a maximum value (for example, 15 minutes) be set for the threshold value T1. This is because a health problem may be considered as a factor for a case where the infant T does not stop crying even after the continuation of holding and walking. However, the threshold value T1 and the maximum value thereof are not limited to the examples described above.

The threshold value IBI1 is a value of the IBI at which holding and walking can be stopped in the crying stopping assistance mode. The threshold value IBI2 is a value of the IBI at which holding and walking can be stopped in the get-to-sleep assistance mode.

The threshold value ISS1 is a value (stage) of the ISS at which holding and walking can be stopped in the crying stopping assistance mode. The threshold value ISS2 is a value (stage) of the ISS at which holding and walking can be stopped in the get-to-sleep assistance mode.

The threshold value SEN1 is a value of the sensor information that is set to determine whether crying of the infant T has been resumed in the crying stopping assistance mode. The threshold value SEN2 is a value of the sensor information that is set to determine whether crying of the infant T has been resumed in the get-to-sleep assistance mode. The threshold value SEN1 and the threshold value SEN2 may be each, for example, a proportion of a vocalization time of the infant T in a unit time.

As described above, the threshold value T2 is a value which is of the length of time elapsed since the stop of holding and walking and which is set to determine whether the infant is in a calm state. For example, the threshold value T2 may be about 30 seconds or may be several minutes according to a childcare environment or the like, and is not limited to these.

As described above, the threshold value T3 is set to determine whether to provide a notification for prompting to check a health condition of the infant T. The threshold value T3 is a preferable maximum value of a total time spent on holding and walking for stopping of crying or for promotion of sleep. For example, it is desirable that the threshold value T3 be set to a value of 10 minutes. However, the threshold value T3 is not limited to the example described above. This is because a health problem may be considered as a factor for a case where the infant T does not stop crying even after the continuation of holding and walking with the infant T.

As described above, the threshold value T4 is set to determine whether to provide a notification for prompting to lay the infant T down to sleep. The threshold value T4 is a value of a preferable length of time elapsed before the infant T is laid down to sleep (the length of time elapsed since the start of holding and sitting). For example, it is desirable that the threshold value T be set to a value of not less than 5 minutes and not more than 8 minutes on the basis of the "Findings that form foundations for the present invention" described above. However, the threshold value T4 is not limited to the example described above.

As described above, the threshold value SEN3 and the threshold value IBI3 are set to determine whether awakening of the infant T is predicted. The threshold value SEN3 can be calculated from the amount of body motion of the infant T while the infant T is awake. The threshold value IBI3 can be calculated from the IBI while the infant T is awake. As described above, the threshold value SEN4 and the threshold value IBI4 are set to determine the possibility that breathing of the infant T has stopped. The threshold value SEN4 can be calculated from the amount of body motion during deep sleep. The threshold value IBI4 may be an upper limit of a range that can be adopted as such a threshold or may be a lower limit thereof.

### <Effect of the present embodiment>

In the present embodiment, notification information for notifying the user U of a childcare behavior to be performed to promote stopping of crying of the infant T or sleep of the infant T is output on the basis of at least one selected from the group consisting of the length of time elapsed since the user U has started holding and walking with the infant T, the IBI, and the ISS. Thus, the user U can perform a childcare behavior on the basis of the notification information and can efficiently promote stopping of crying of the infant T or sleep of the infant T.

In addition, in the present embodiment, at least one selected from the group consisting of the IBI and the ISS since the user U has started holding and walking with the infant T is output in real time. Therefore, the user U can quantitatively understand a change in the state of the infant T by holding and walking and can reflect such feedback in a childcare behavior to be performed to promote stopping of crying of the infant T or sleep of the infant T by, for example, contriving a way to hold the child T.

Here, the effect of the present embodiment will be described in comparison with the system disclosed in Patent Literature 1 described above. The system disclosed in Patent Literature 1 is assumed to be used in a method of detecting a drowsy state before sleep and, at that timing, letting a caregiver pull out a pacifier from the mouth of an infant. As described in Patent Literature 1 as well, a pacifier may cause a risk of aspiration for infants during sleep. In addition, there is a possibility that a sensor in the pacifier may break down or a possibility that a caregiver may fail to perform an operation of pulling out the pacifier from the infant in a drowsy period before sleep. Therefore, using the system disclosed in Patent Literature 1 to get an infant to sleep is not preferable or requires extreme caution. In addition, as described above, it is difficult for a crying infant to use a pacifier. Further, some infants do not like a pacifier. Therefore, the use of the system disclosed in Patent Literature 1 does not reduce the stress of a caregiver that goes on until an infant stops crying or gets to sleep.

In comparison with the system disclosed in Patent Literature 1, the present embodiment enables assistance of a childcare behavior of a caregiver at every phase from stopping of crying of infants to promotion of sleep of infants.

### [First variation]

A first variation in which the above-described embodiment is altered will be described. In an information processing system 1 in accordance with the first variation, the processor 11 (notification unit 105) outputs notification information on the basis of information acquired from an assistance model. The assistance model is a model that receives an input of at least one selected from the group consisting of the elapsed time, the IBI, and the ISS, and outputs a success rate of a childcare behavior of the user U based on the notification information. The other points are configured in the same manner as the above-described embodiment.

Here, the assistance model is constituted by, for example, a neural network that handles time-series data (specifically, recurrent neural network (RNN), long short term memory (LSTM), or the like). In this case, time-series data of the IBI and time-series data of the ISS are input to the assistance model. However, the assistance model may be constituted by another neural network, a machine learning algorithm other than the neural network, or a rule-based model other than machine learning.

Further, the "success rate of a childcare behavior of the user U based on the notification information" is a probability that the infant T will go into a desirable state in a case where the user U performs a childcare behavior on the basis of the notification information from the information processing system 1. Hereinafter, the "success rate of a childcare behavior of the user U based on the notification information" is also referred to simply as "success rate of the notification information". In addition, the desirable state is, for example, a "state in which crying is not resumed for a predetermined period of time", but is not limited to this state.

As a more specific example, the assistance model includes a first assistance model, a second assistance model, and a third assistance model. The first assistance model outputs a success rate of the notification information N2 (first notification information). The second assistance model outputs a success rate of the notification information N5 (second notification information). The third assistance model outputs a success rate of the notification information N6 (third notification information). Note that each assistance model is generated with use of training data. Details of the training data will be described in a second variation, which will be described later.

In the present variation, the operation of the information processing system 1 is altered as below. The other steps are as described above.

In step S105 in Fig. 6, in addition to calculating the IBI and the ISS, the processor 11 inputs the time-series data of the calculated IBI and the time-series data of the calculated ISS to the first assistance model, the second assistance model, and the third assistance model.

Further, in step S201 in Figs. 9 and 10, the processor 11 determines whether the success rate output from the first assistance model exceeds a threshold value.

Further, in step S204 in Figs. 9 and 10, the processor 11 determines whether the success rate output from the second assistance model exceeds a threshold value.

Further, in step S210 in Figs. 9 and 10, the processor 11 determines whether the success rate output from the third assistance model exceeds a threshold value.

As described above, the information processing system 1 in the present variation outputs the notification information in a case where the success rate output from the assistance model exceeds the threshold value, and thus makes it possible to further increase the success rate of the notification information.

### [Second variation]

A second variation in which the above-described first variation is further altered will be described. In the information processing system 1 in accordance with the second variation, the processor 11 (notification unit 105) outputs notification information with reference to information acquired from a modified assistance model. The modified assistance model is a model obtained by making a modification to the above-described modified model with use of the previously accumulated following information (i) and (ii): (i) information indicating success or failure of a childcare behavior based on the notification information; and (ii) at least one selected from the group consisting of the length of time elapsed before the output of the notification information, the heart rate information, and the infant state score. For example, the processor 11 accumulates the information indicating success or failure of the childcare behavior based on the notification information and at least one selected from the group consisting of the length of time elapsed before the output of the notification information, the IBI, and the ISS. In addition, the processor 11 modifies the assistance model with use of the accumulated information. The other points are configured in the same manner as the first variation.

Specifically, the processor 11 acquires the information indicating success or failure of the notification information on the basis of the operation of the user U at any point in time after the notification information has been output. For example, in a case where the user U has stopped holding and walking on the basis of the notification information N2, and then crying of the infant T has been resumed, the user U performs an operation of inputting information indicating "failure". Further, for example, in a case where the user U has started holding and sitting on the basis of the notification information N5, and then crying of the infant T has not been resumed, the user U performs an operation of inputting information indicating "success". Further, for example, in a case where the user U has laid the infant T down to sleep on the basis of the notification information N6, and then crying of the infant T has been resumed, the user U performs an operation of inputting information indicating "failure".

The processor 11 modifies the first assistance model by retraining with use of training data in which the length of a period of time elapsed from the output of the notification information N 1 to the output of the notification information N2, the IBI and the ISS during such a period of time, and the information indicating success or failure of the notification information N2 are associated with each other.

Further, the processor 11 modifies the second assistance model by retraining with use of training data in which the length of a period of time elapsed from the output of the notification information N2 to the output of the notification information N5, the IBI and the ISS during such a period of time, and the information indicating success or failure of the notification information N5 are associated with each other.

Further, the processor 11 modifies the third assistance model by retraining with use of training data in which the length of a period of time elapsed from the output of the notification information N5 to the output of the notification information N6, the IBI and the ISS during such a period of time, and the information indicating success or failure of the notification information N6 are associated with each other.

Such modification to the assistance model may be carried out for each infant T. Thus, the information processing system 1 can output the notification information at a timing at which a higher success rate is achieved according to a target infant T.

In addition, such modification to the assistance model may be carried out with use of training data obtained from a plurality of infants T. Thus, the information processing system 1 can output the notification information at a timing at which a higher success rate is achieved even in a state in which training data is not sufficiently accumulated for the target infant T.

In addition, such modification to the assistance model may be carried out with use of training data obtained from a plurality of infants T having similar attributes. The attribute may be, for example, an age in month, a sibling composition, a room environment, a gender, an attribute of the user U, and the like, but is not limited thereto. Even in a state in which the training data is not sufficiently accumulated for the target infant T, it is possible to output the notification information at a timing at which a higher success rate is achieved.

### [Other variations]

### (Variation of system configuration)

Further, in the present embodiment, an example in which the information processing system 1 includes the user terminal 10, the wearable terminal 20, and the audio output device 30 has been described.

The information processing system 1 may further include a server. The server may be, for example, a server disposed in a cloud. In this case, the server may perform the process of calculating the IBI and the ISS. Further, the server may perform the process of determining whether to output the notification information on the basis of at least one selected from the group consisting of the elapsed time, the IBI, and the ISS.

Further, the information processing system 1 may include an external display such as a television. In this case, the processor 11 may set an external display instead of or in addition to the audio output device 30 as the output destination to which the IBI and the ISS are output in real time.

Further, the information processing system 1 may include a wearable terminal (e.g., a smart watch) for the user U instead of or in addition to the user terminal 10.

Further, the user terminal 10 is not limited to the smartphone described above, and may be a tablet, a notebook personal computer, a desktop personal computer, a smart speaker, or other type of computer. Further, the user terminal 10 may include the audio output device 30. However, the user terminal 10 only needs to include at least the processor 11, the memory 12, and the network interface 13, and one or some or all of the input device, the display device, and the audio output device may be external devices.

### (Variation of IBI)

In the above-described embodiment, an example in which the IBI is used as the heart rate information has been described. However, the heart rate information only needs to be information on a heart rate, and may be information other than the IBI.

### (Variation of ISS)

In the above-described embodiment, an example in which seven stages of the ISS are used has been described. However, the number of stages is not limited to seven. Further, the theoretical values of the ISS may be used as they are.

In addition, according to a situation, a higher priority may be given to the theoretical values of the ISS than to the seven stages of the ISS. For example, the calculation unit 103 may calculate the seven stages of the ISS in a case where the sensor information is in agreement with the theoretical values of the ISS, and may use the theoretical values of the ISS in a case where the sensor information is not in agreement with the theoretical values of the ISS. For example, there is a high possibility that the sensor information from the body motion sensor 25 is not in agreement with the theoretical values of the ISS in, for example, a situation in which the caregiver is holding and walking with the infant T. In addition, there is a high possibility that the sensor information from the vocalization sensor 26 is not in agreement with the theoretical values of the ISS in, for example, a situation in which an environmental sound (e.g., a sound from a television and a rubbing sound of clothing). As described above, with use of one of the seven stages of the ISS and the theoretical values of the ISS according to a situation, the ISS representing the state of the infant T can be used more accurately.

### (Variation of sensor)

Further, in the above-described embodiment, an example in which the pulse wave sensor 24 is used as the sensor that detects the heart rate information has been described. However, the sensor that detects the heart rate information may be another sensor (for example, an electrocardiogram holter) or the like.

In addition, in the above-described embodiment, an example in which the wearable terminal 20 includes various sensors has been described. The wearable terminal 20 is not limited to such a configuration. Alternatively, a sensor that detects the state of the infant T may be a sensor which is installed outside the wearable terminal 20. Examples of such a sensor include, but not limited to, a sensor which is installed in a room for taking care of the infant T, a sensor which a user U wears, and the like. In this case, the processor 11 may directly communicate with the external sensor to acquire the sensor information.

### (Variation of sensor information)

Further, in the above-described embodiment, an example in which the sensor information includes information indicating a pulse wave, a body motion, and vocalization has been described. In addition to these, the sensor information may include information indicating opening and closing of eyes. For example, the information indicating opening and closing of eyes can be detected by a ring-type sensor worn on the finger of the user U. In this case, the user U operates the ring-type sensor when the user U checks opening and closing of eyes of the infant T. When the ring-type sensor has been operated, the processor 11 determines that opening or closing of the eye has been detected. Further, for example, the sensor information may include information indicating a body temperature, skin conductance, or oxygen partial pressure. These pieces of sensor information can be detected by, for example, a body temperature sensor, a skin conductance sensor, or an oxygen partial pressure sensor. Note that the sensor information is not limited to these, and may include other information indicating the state of the infant T.

### (First variation of real-time output)

Further, in the above-described embodiment, an example in which the change-over-time information D5 indicating changes over time in the IBI and the ISS has been described. In addition to this, the processor 11 may output a prediction value obtained by prediction from changes over time in the IBI and the ISS. As a technique for calculating a prediction value from changes over time in time-series data, a known technique can be employed. Thus, the user U can know about a change of the state of the infant T to be predicted in the future (e.g., it appears that crying of the infant T will be resumed or it appears that the infant T will be calmed down) while holding and walking (or holding and sitting) with the infant T.

### (Second variation of real-time output)

In the above-described embodiment, an example in which a graph representing a time axis as the horizontal axis and representing the IBI and the ISS as the vertical axis is used as a form of a real-time output of the IBI and the ISS has been described. The real-time output of the IBI and the ISS is not limited to this. The processor 11 may use other form of output as the form of the output. For example, the processor 11 may display, on the touch panel 14, a predetermined region that is filled with a color associated with each stage of the ISS. Further, for example, the processor 11 may display, on the touch panel 14, an icon (e.g., a face mark) associated with each stage of the ISS. Further, for example, the processor 11 may output, to the audio output device 30, a ring tone with a pattern associated with each stage of the ISS.

### (Variation of notification information)

Further, the notification information output by the information processing system 1 is not limited to the notification information N1 to the notification information N8 described above, and may include other notification information.

For example, the information processing system 1 may output notification information for providing a notification of tips for holding and walking. The notification information includes, for example, the following information: (i) the caregiver brings the infant T into contact with the body of the caregiver and supports the infant T with hands to prevent the head of the infant T from wobbling; (ii) the caregiver increases the area in which the abdomen of the infant T closely contacts with the body of the caregiver; (iii) the caregiver utilizes a baby sling, a baby holder, a sling, or the like in order to reduce the burden on the caregiver: (iv) since the body temperature of the infant T tends to increase, the caregiver makes the infant T wear slightly light clothes in hot weather by, for example, removing socks from the infant T; (v) the caregiver walks in a tidy place with no steps (e.g., corridor); and (vi) the caregiver walks at a constant pace in a manner that the number of changes of the direction and the like is as small as possible.

Further, for example, the information processing system 1 may output notification information for providing a notification of a prediction time before the notification information N1, N2, or N6 is output. The notification information may be, for example, audio information saying, for example, "Since the infant is 80% sleeping", please walk for another one minute", but is not limited thereto.

Further, for example, in a case where the information processing system 1 has determined that the state of the infant T tends to be changed into a more preferable state (e.g., a reduction of crying, an increase in the IBI, and the like) before the notification information N1, N2, or N6 is output, the information processing system 1 may output notification information for cheering up the user U. The notification information may be, for example, audio information saying, for example, "since the infant will stop crying soon, please go for it.", but is not limited thereto.

### (Variation of output destination of notification information)

The output destination of the notification information is not limited to the audio output device 30. For example, the processor 11 may display the notification information on the touch panel 14. Further, for example, the processor 11 may output the notification information by displaying the notification information on the wearable terminal for the user U or by vibrating the wearable terminal for the user U.

### (Variation of storage function)

Further, the present embodiment may have a storage function of storing the sensor information acquired in each mode and the information indicating success or failure of a childcare behavior. A location in which these pieces of information are stored may be the memory 12 of the user terminal 10 or may be a cloud storage. Accordingly, the present embodiment may have a function of browsing and printing the stored information.

### (Variation having arbitrary recording mode)

The present embodiment may further include an arbitrary recording mode in which at least one selected from the group consisting of the sensor information, the IBI, and the ISS is recorded in any situation. In the arbitrary recording mode, the processor 11 acquires the sensor information during a period from a start base on an instruction performed by an operation of the user U to an end based thereon, and calculates the IBI or the ISS. Further, the processor 11 stores at least one selected from the group consisting of the sensor information, the IBI, and the ISS as recording information. Further, the processor 11 may store a comment input by the user U or a timing marker in association with the recording information.

Thus, the user U can check the state of the infant T in any situation. For example, by utilizing the arbitrary recording mode, the user U can check on what kind of music is listened to by the infant T when the infant T is calm, which of the places, a futon and a bed, is a place where the infant T can sleep better, and the like.

### (Variation of mode selection)

Further, it has been described that the present embodiment includes the crying stopping assistance mode, the get-to-sleep assistance mode, and the awakening prediction mode. However, the present embodiment is not limited to including all of these modes and may include one or some of the modes.

### [Software or hardware implementation example]

In the above-described embodiment, an example has been described in which the function of the information processing system 1 is realized by a program that is stored in a memory of each of the devices constituting the information processing system 1 and that is a program (software) for causing a processor of each of the devices to execute the above-described steps.

The program may be stored in at least one non-transitory computer-readable storage medium. This storage medium may be or may not be included in the above devices. In the latter case, the program may be supplied to the devices via any wired or wireless transmission medium.

Note that what realizes the functional blocks (the sensor information acquisition unit 101, the start information acquisition unit 102, the calculation unit 103, the output unit 104, and the notification unit 105) of the information processing system 1 is not limited to the above-described program. Further, one or some or all of the above-described functional blocks may be realized by a logic circuit (hardware). Further, an integrated circuit in which such a logic circuit is formed is included in the scope of the present invention. Other than those, for example, it is possible to realize the above function by a quantum computer.

The present invention is not limited to the embodiments, but can be altered by a skilled person in the art within the scope of the claims. The present invention also encompasses, in its technical scope, any embodiment derived by combining technical means disclosed in differing embodiments.

### [Summation]

An information processing system in accordance with a first aspect of the present embodiment includes: a sensor information acquisition unit that acquires sensor information from a sensor that detects a state of an infant; a calculation unit that calculates, on a basis of the sensor information, at least one selected from the group consisting of heart rate information related to a heart rate of the infant and an infant state score indicating any one of states of the infant from a wailing state to a sleep state; an output unit that outputs, in real time, information indicating at least one selected from the group consisting of the heart rate information and the infant state score; and a notification unit that outputs notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

The above-described configuration enables the user to, after having started holding and walking with a crying infant, carry out a childcare behavior to be performed to promote stopping of crying of the infant or sleep of the infant in accordance with output of the notification information. Thus, it is possible to efficiently promote stopping of crying of the infant or sleep of the infant. Further, as a result, user's stress is reduced.

An information processing system in accordance with a second aspect of the present embodiment is configured, in the first aspect, to further include a start information acquisition unit that acquires information indicating that the user has started holding the infant and walking with the infant, wherein the notification information includes first notification information for providing a notification to prompt stopping of the holding and walking.

The above-described configuration enables the user to, at an appropriate timing, stop holding and walking for promoting stopping of crying of the infant or sleep of the infant in accordance with output of the first notification information.

An information processing system in accordance with a third aspect of the present embodiment is configured, in the second aspect, such that the notification information includes second notification information for providing a notification to prompt start of holding and sitting, and the notification unit that outputs the second notification information on the basis of at least one selected from the group consisting of a length of time elapsed since output of the first notification information, the heart rate information, and the infant state score.

The above-described configuration enables the user to, at an appropriate timing, start holding and sitting for promoting sleep of the infant in accordance with output of the second notification information.

An information processing system in accordance with a fourth aspect of the present embodiment is configured, in the third aspect, such that the notification information further includes third notification information for providing a notification to prompt the user to lay the infant down to sleep, and the notification unit that outputs the third notification information on the basis of at least one selected from the group consisting of a length of time elapsed since output of the second notification information, the heart rate information, and the infant state score.

The above-described configuration enables the user to, at an appropriate timing, lay the infant down to sleep for promoting sleep of the infant in accordance with output of the third notification information.

An information processing system in accordance with a fifth aspect of the present embodiment is configured, in any one of the first to fourth aspects, such that the notification information further includes fourth notification information for providing a notification to prompt the user to check a health condition of the infant, and the notification unit outputs the fourth notification information in a case where a total time spent on the holding and walking exceeds a threshold value.

The above-described configuration enables the user to pay attention to a health condition of the infant in a case where stopping of crying or sleep is not promoted by repeating holding and walking.

An information processing system in accordance with a sixth aspect of the present embodiment is configured, in any one of the first to fifth aspects, such that the notification unit outputs the notification information with reference to information acquired from an assistance model, and the assistance model is a model that: receives an input of at least one selected from the group consisting of the elapsed time, the heat rate information, and the infant state score; and outputs a success rate of a childcare behavior of the user based on the notification information.

The above-described configuration enables provision, to the user, of a notification of a childcare behavior to be performed to promote stopping of crying of the infant or sleep of the infant at a timing based on a success rate.

An information processing system in accordance with a seventh aspect of the present embodiment is configured, in the sixth aspect, such that the notification unit refers to information acquired from the assistance model that has been modified on the basis of: information indicating success or failure of the childcare behavior based on the notification information; and at least one selected from the group consisting of the length of time elapsed before output of the notification information, the heart rate information, and the infant state score.

The above-described configuration enables feedback on success or failure of a childcare behavior based on the notification information to be reflected in improving the accuracy of a modified model.

An information processing system in accordance with an eighth aspect of the present embodiment is configured, in any one of the first to seventh aspects, such that the sensor includes a sensor that detects at least one selected from the group consisting of a pulse wave of the infant, a body motion thereof, and vocalization thereof.

The above-described configuration enables the state of the infant to be detected on the basis of a pulse wave, a body motion, and vocalization.

An information processing method in accordance with a ninth aspect of the present embodiment includes: a sensor information acquisition step of acquiring sensor information from a sensor that detects a state of an infant; a calculation step of calculating, on a basis of the sensor information, at least one selected from the group consisting of heart rate information related to a heart rate of the infant and an infant state score indicating any one of states of the infant from a wailing state to a sleep state; an output step of outputting, in real time, information indicating at least one selected from the group consisting of the heart rate information and the infant state score; and a notification step of outputting notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

The above-described configuration brings about an effect that is similar to the effect brought about by the first aspect.

A program in accordance with a tenth aspect of the present embodiment is a program for causing a computer to function as an information processing system according to any one of claims 1 to 8, the program causing the computer to function as each of the foregoing units.

Furthermore, part or whole of the above-described embodiment can also be expressed as follows: an information processing system comprising at least one processor, the at least one processor carrying out: a sensor information acquisition process of acquiring sensor information from a sensor that detects a state of an infant; a calculation process of calculating, on a basis of the sensor information, at least one selected from the group consisting of heart rate information related to a heart rate of the infant and an infant state score indicating any one of states of the infant from a wailing state to a sleep state; an output process of outputting, in real time, information indicating at least one selected from the group consisting of the heart rate information and the infant state score; and a notification process of outputting notification information for notifying a user of a childcare behavior to be performed to promote stopping of crying of the infant or sleep of the infant on a basis of at least one selected from the group consisting of the length of time elapsed since the user has started holding the infant and walking with the infant, the heart rate information, and the infant state score. The above-described configuration brings about an effect that is similar to the effect brought about by the first aspect.

The above-described configuration brings about an effect that is similar to the effect brought about by the first aspect.

### Reference Signs List

1: information processing system
10: user terminal
20: wearable terminal
11, 21: processor
12, 22: memory
13, 23: network interface
14: touch panel
24: pulse wave sensor
25: body motion sensor
26: sensor
26: vocalization sensor
30: audio output device

## Claims

1. An information processing system comprising:
a sensor information acquisition unit that acquires sensor information from a sensor that detects a state of an infant;
a calculation unit that calculates, on a basis of the sensor information, at least one selected from the group consisting of heart rate information related to a heart rate of the infant and an infant state score indicating any one of states of the infant from a wailing state to a sleep state;
an output unit that outputs, in real time, information indicating at least one selected from the group consisting of the heart rate information and the infant state score; and
a notification unit that outputs notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

2. The information processing system according to claim 1, further comprising a start information acquisition unit that acquires information indicating that the user has started holding the infant and walking with the infant,
wherein the notification information includes first notification information for providing a notification to prompt stopping of the holding and walking.

3. The information processing system according to claim 2, wherein
the notification information includes second notification information for providing a notification to prompt start of holding and sitting, and
the notification unit that outputs the second notification information on the basis of at least one selected from the group consisting of a length of time elapsed since output of the first notification information, the heart rate information, and the infant state score.

4. The information processing system according to claim 3, wherein
the notification information further includes third notification information for providing a notification to prompt the user to lay the infant down to sleep, and
the notification unit that outputs the third notification information on the basis of at least one selected from the group consisting of a length of time elapsed since output of the second notification information, the heart rate information, and the infant state score.

5. The information processing system according to claim 1 or 2, wherein
the notification information further includes fourth notification information for providing a notification to prompt the user to check a health condition of the infant, and
the notification unit outputs the fourth notification information in a case where a total time spent on the holding and walking exceeds a threshold value.

6. The information processing system according to claim 1 or 2, wherein
the notification unit outputs the notification information with reference to information acquired from an assistance model, and
the assistance model is a model that:
receives an input of at least one selected from the group consisting of the elapsed time, the heat rate information, and the infant state score; and
outputs a success rate of a childcare behavior of the user based on the notification information.

7. The information processing system according to claim 6, wherein
the notification unit refers to information acquired from the assistance model that has been modified on the basis of:
information indicating success or failure of the childcare behavior based on the notification information; and
at least one selected from the group consisting of the length of time elapsed before output of the notification information, the heart rate information, and the infant state score.

8. The information processing system according to claim 1 or 2, wherein
the sensor includes a sensor that detects at least one selected from the group consisting of a pulse wave of the infant, a body motion thereof, and vocalization thereof.

9. An information processing method comprising:
a sensor information acquisition step of acquiring sensor information from a sensor that detects a state of an infant;
a calculation step of calculating, on a basis of the sensor information, at least one selected from the group consisting of heart rate information related to a heart rate of the infant and an infant state score indicating any one of states of the infant from a wailing state to a sleep state;
an output step of outputting, in real time, information indicating at least one selected from the group consisting of the heart rate information and the infant state score; and
a notification step of outputting notification information for notifying a user of a childcare behavior to be performed to promote stopping of crying of the infant or sleep of the infant on a basis of at least one selected from the group consisting of a length of time elapsed since the user has started holding the infant and walking with the infant, the heart rate information, and the infant state score.

10. A program for causing a computer to function as an information processing system according to claim 1 or 2, the one or more programs causing the computer to function as each of the foregoing units.
